# EUROPEAN PATENT SPECIFICATION

(11) **EP 2 698 109 B1**
(45) Date of publication and mention of the grant of the patent: **04.11.2015**
(21) Application number: 13178976.0
(22) Date of filing: 01.08.2013
(51) Int. Cl.: A61B 5/055, G01R 33/28, H04N 5/225, A61B 5/00, G01R 33/48, G01R 33/38, G01R 33/383

(54) **MRI apparatus combined with lightfield camera**
Mit Lichtfeldkamera kombinierte MRT-Vorrichtung
Appareil d'IRM combiné avec un caméra de champ lumineux

(30) Priority: 15.08.2012 IL 22149112
(43) Date of publication of application: 19.02.2014
(73) Proprietor: Aspect Imaging Ltd., 60850 Shoham (IL)
(72) Inventor: Rapoport, Uri, 73115 Moshav Ben Shemen (IL); Batt, Aryeh, 90440 Har Hevron (IL)
(74) Representative: Lecomte & Partners

(56) References cited:
- WO-A1-2009/129457
- WO-A1-2011/100993
- JP-A- H10 165 393
- US-A1- 2009 295 829

## Description

### FIELD OF THE INVENTION

The present invention relates to an MRI apparatus and, more specifically, to MRI apparatus provided with a light-field camera.

### BACKGROUND OF THE INVENTION

Magnetic resonance imaging (MRI) is a medical imaging technique used in radiology to visualize internal structures of the body in detail. MRI makes use of the property of nuclear magnetic resonance (NMR) to image nuclei of atoms inside the body.

An MRI scanner is a device in which the patient lies within a large, powerful magnet where the magnetic field is used to align the magnetization of some atomic nuclei in the body, and radio frequency pulse is used to alter the alignment of this magnetization. This causes the nuclei to precess as the magnetization returns to equilibrium, thereby producing a radio frequency signal related to the local magnetic field experienced by each nucleus. This information is recorded to construct an image of the scanned area of the body. Magnetic field gradients cause nuclei at different locations to precess at different speeds. By using gradients in different directions 2D images or 3D volumes can be obtained in any arbitrary orientation.

JP10165393A discloses an MRI apparatus provided with luminescence means composed of a plurality of illuminants arranged within the MRI apparatus. An MRI image is combined with a luminescence image to improve the diagnostic capabilities of the MRI apparatus. Document WO2009/129457 represents the closest prior art disclosing an MRI apparatus for obtaining MRI and optical images concurrently.

A light-field camera (plenoptic) camera is a camera that uses a microlens array to capture 4D light field information about a scene. Such light field information can be used to improve the solution of computer graphics and vision-related problems.

US 8189065 discloses a method and apparatus for full-resolution light-field capture and rendering. A radiance camera is described in which the microlenses in a microlens array are focused on the image plane of the main lens instead of on the main lens, as in conventional plenoptic cameras. The microlens array may be located at distances from the photosensor greater than the focal length f of the microlenses. Radiance cameras in which the distance of the microlens array from the photosensor is adjustable, and in which other characteristics of the camera are adjustable, are described. Digital and film embodiments of the radiance camera are described. A full-resolution light-field rendering method may be applied to flats captured by a radiance camera to render higher-resolution output images than are possible with conventional plenoptic cameras and rendering methods.

The MRI equipment is provided with means for creating a 3D image of an object of interest and any cross sectional view. In this connection, a combination of an MR image and an optical image looks very promising. Thus, there is a long-felt and unmet need for providing an MRI apparatus which is able to combine an MR image and an optical image such that the aforesaid images are taken concurrently and parallax angles and field depths of the images are identical.

### SUMMARY OF THE INVENTION

It is hence one object of the invention to disclose an MRI apparatus configured for inducing a magnetic resonance signal from an object to be imaged. The aforesaid apparatus comprises a magnet poles for creating a homogeneous magnetic field; and a set of RF coils for generating an RF excitation pulse in the imaging volume of the apparatus, and for acquiring magnetic resonance signals which are due to the RF excitation pulse.

It is a core purpose of the invention to provide the apparatus with a light-field (plenoptic) camera such that the object is imaged concurrently by MRI and plenoptic channels and images obtained from the two channels are superimposed.

Another object of the invention is to disclose the MRI and plenoptic images characterized by an identical parallax angle.

A further object of the invention is to disclose the MRI and plenoptic images characterized by an identical depth of field.

A further object of the invention is to disclose a method of MR imaging by means of inducing a magnetic resonance signal from an object to be imaged. The aforesaid method comprises the steps of (a) providing a MRI apparatus configured for inducing a magnetic resonance signal from an object to be imaged, the apparatus comprising: (i) magnet poles for creating a homogeneous magnetic field; and (ii) a set of RF coils for generating an RF excitation pulse in the imaging volume of the apparatus, and for acquiring magnetic resonance signals which are due to the RF excitation pulse; (b) concurrently imaging the object; (c) rendering MR and plenoptic images; and (d) superimposing the MR and plenoptic images.

It is a further object of the present invention to disclose an MRI apparatus for providing superimposed MRI and plenoptic images, characterized in that said apparatus comprises: magnet poles for creating a homogeneous magnetic field; a set of RF coils for generating an RF excitation pulse in the imaging volume of the apparatus, and for acquiring magnetic resonance signals which are due to the RF excitation pulse; MR imaging means for converting said magnetic resonance signals into a magnetic resonance image; a light-field (plenoptic) camera configured to obtain plenoptic images concurrently with said magnetic resonance images; and, superposition means for superimposing said magnetic resonance and plenoptic images.

It is a further object of the present invention to disclose the MRI apparatus as defined in any of the above, characterized in that said magnetic resonance images and said plenoptic images are characterized by an identical parallax angle.

It is a further object of the present invention to disclose the MRI apparatus as defined in any of the above, characterized in that said magnetic resonance images and said plenoptic images are characterized by an identical depth of field.

It is a further object of the present invention to disclose the MRI apparatus as defined in any of the above, characterized in that said magnet poles are constructed from permanent magnets.

It is a further object of the present invention to disclose a method for providing superimposed magnetic resonance and plenoptic images, comprising: providing an MRI apparatus as defined in any of the above; placing an object within a field of view of said MRI apparatus; using said MR imaging means to produce a magnetic resonance image characterized by a parallax angle and a depth of field; using said plenoptic camera to produce, concurrently with said magnetic resonance image, a plenoptic image characterized by a parallax angle and a depth of field; rendering magnetic resonance and plenoptic images; and, superimposing said magnetic resonance and plenoptic images.

It is a further object of the present invention to disclose such a method, characterized in that said step of producing a plenoptic image comprises producing a plenoptic image having a parallax angle identical to said parallax angle of said magnetic resonance image.

It is a further object of the present invention to disclose the method as defined in any of the above, characterized in that said step of producing a plenoptic image comprises producing a plenoptic image having a depth of field identical to said depth of field of said magnetic resonance image.

It is a further object of the present invention to disclose the method as defined in any of the above, characterized in that said step of providing an MRI apparatus comprises providing an MRI apparatus comprising pole pieces constructed from permanent magnets.

### BRIEF DESCRIPTION OF THE DRAWINGS

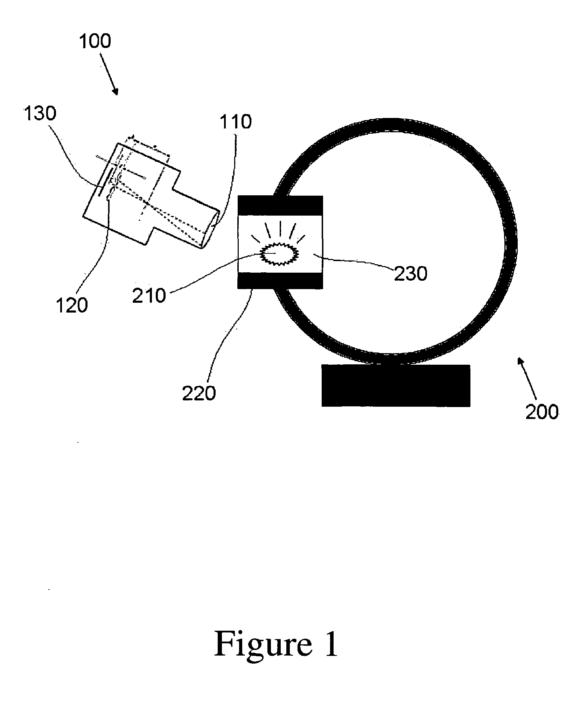

In order to understand the invention and to see how it may be implemented in practice, a plurality of embodiments is adapted to now be described, by way of non-limiting example only, with reference to the accompanying drawing, in which **Fig. 1** is a schematic view of an MRI apparatus a light-field (plenoptic) camera.

### DETAILED DESCRIPTION OF THE INVENTION

The following description is provided so as to enable any person skilled in the art to make use of said invention and sets forth the best modes contemplated by the inventor of carrying out this invention. Various modifications, however, are adapted to remain apparent to those skilled in the art, since the generic principles of the present invention have been defined specifically to provide an MRI apparatus configured for inducing a magnetic resonance signal from an object to be imaged and a method of using the same.

Reference is now made to **Fig. 1**, presenting a schematic view of an MRI apparatus of the present invention. The magnetic portion 200 is configured for inducing a magnetic resonance signal from an object to be imaged. Magnet poles 220 create a homogeneous magnetic field. A set of RF coils (not shown) generates an RF excitation pulse in the imaging volume 230 of the apparatus and acquires magnetic resonance signals from a sample 210 to be examined which are due to the RF excitation pulse. A plenoptic camera 100 is oriented for receiving optical radiation (i.e. radiation of wavelength shorter than microwave) emitted or reflected or scattered by the sample 210. Detection of bioluminescent or fluorescent radiation is in the scope of the present invention.

The plenoptic camera 100 comprises a main lens 110 and a microlens array 120. The aforesaid array 120 focuses a matrix of micro images on an image detector (CCD) 130.

Software known in the art is able to refocus an image obtained by the plenoptic camera and to change a parallax angle (point of view). This feature of the proposed technical solution provides an opportunity of fine adjustment of MR and plenoptically rendered images. The images examined in association with each other improve diagnostic capabilities.

In one embodiment of the present invention, it comprises an MRI apparatus configured for inducing a magnetic resonance signal from an object to be imaged is disclosed. The aforesaid apparatus comprises magnet poles for creating a homogeneous magnetic field and a set of RF coils for generating an RF excitation pulse in the imaging volume of the apparatus and for acquiring magnetic resonance signals which are due to the RF excitation pulse. In some embodiments of the invention, the magnet poles are those of a permanent magnet or an electromagnet.

It is a core feature of the present invention to provide the apparatus with a light-field (plenoptic) camera such that said object is imaged concurrently by MRI and plenoptic channels and the MRI and plenoptic images are superimposed. In preferred embodiments, the apparatus is provided with software known in the art that performs the superposition of the two images.

In some embodiments of the apparatus, the MRI and plenoptic images are characterized by an identical parallax angle.

In some embodiments of the apparatus, the MRI and plenoptic images are characterized by an identical depth of field. The depth of field can be chosen manually by the operator or set automatically by the apparatus.

In accordance with another embodiment of the present invention, a method of MR imaging by means of inducing a magnetic resonance signal from an object to be imaged is disclosed. The aforesaid method comprises the steps of (a) providing a MRI apparatus configured for inducing a magnetic resonance signal from an object to be imaged, said apparatus comprising: (i) a magnet poles for creating a homogeneous magnetic field; and (ii) a set of RF coils for generating an RF excitation pulse in the imaging volume of the apparatus and for acquiring magnetic resonance signals which are due to the RF excitation pulse; (b) concurrently imaging said object; (c) rendering MR and plenoptic images; and (d) superimposing said MR and plenoptic images. The MR imaging can be performed by using any type of MR imaging instrument known in the art. In particular, MR imaging instruments in which the magnetic field is produced by a permanent magnet are considered by the inventor to be within the scope of the invention.

## Claims

1. An MRI apparatus for providing superimposed MRI and plenoptic images, wherein said apparatus comprises:
magnet poles (220) for creating a homogeneous magnetic field;
a set of RF coils for generating an RF excitation pulse in the imaging volume (230) of the apparatus, and for acquiring magnetic resonance signals which are due to the RF excitation pulse;
MR imaging means for converting said magnetic resonance signals into a magnetic resonance image;
a light-field, i.e. plenoptic, camera (100) configured to obtain the plenoptic images concurrently with said magnetic resonance images; and,
superposition means for superimposing said magnetic resonance and plenoptic images.

2. The MRI apparatus according to claim 1, wherein said magnetic resonance images and said plenoptic images are **characterized by** an identical parallax angle.

3. The MRI apparatus according to claim 1, wherein said magnetic resonance images and said plenoptic images are **characterized by** an identical depth of field.

4. The MRI apparatus according to claim 1, wherein said magnet poles (220) are constructed from permanent magnets.

5. A method for providing superimposed magnetic resonance and plenoptic images, wherein said method comprises:
providing an MRI apparatus according to claim 1;
placing an object within a field of view of said MRI apparatus;
using said MR imaging means to produce a magnetic resonance image **characterized by** a parallax angle and a depth of field;
using said plenoptic camera to produce, concurrently with said magnetic resonance image, a plenoptic image **characterized by** a parallax angle and a depth of field;
rendering magnetic resonance and plenoptic images; and,
superimposing said magnetic resonance and plenoptic images.

6. The method according to claim 5, wherein said step of producing a plenoptic image comprises producing a plenoptic image having a parallax angle identical to said parallax angle of said magnetic resonance image.

7. The method according to claim 5, wherein said step of producing a plenoptic image comprises producing a plenoptic image having a depth of field identical to said depth of field of said magnetic resonance image.

8. The method according to claim 5, wherein said step of providing an MRI apparatus comprises providing an MRI apparatus comprising magnet poles constructed from permanent magnets.

## Patentansprüche

1. MRT-Vorrichtung zur Bereitstellung überlagerter MRT- und plenoptischer Abbildungen, wobei besagte Vorrichtung umfasst.
Magnetpole (220) zur Erzeugung eines homogenen Magnetfelds;
einen Satz von Hochfrequenzspulen zur Erzeugung eines Hochfrequenz-Anregungsimpulses in dem Abbildungsvolumen (230) der Vorrichtung und zur Akquirierung von Magnetresonanzsignalen, die auf den Hochfrequenz-Anregungsimpuls zurückzuführen sind;
Magnetresonanz-Abbildungsmittel zur Umwandlung besagter Magnetresonanzsignale in eine Magnetresonanzabbildung;
eine Lichtfeld-, d.h. plenoptische, Kamera (100), die dazu gestaltet ist, die plenoptischen Abbildungen zeitgleich mit besagten Magnetresonanzabbildungen zu erhalten; und
Überlagerungsmittel zur Überlagerung besagter Magnetresonanz- und plenoptischer Abbildungen.

2. MRT-Vorrichtung nach Anspruch 1, wobei die Magnetresonanzabbildungen und die plenoptischen Abbildungen durch einen identischen Parallaxenwinkel gekennzeichnet sind.

3. MRT-Vorrichtung nach Anspruch 1, wobei die Magnetresonanzabbildungen und die plenoptischen Abbildungen durch eine identische Feldtiefe gekennzeichnet sind.

4. MRT-Vorrichtung nach Anspruch 1, wobei die Magnetpole (220) aus Permanentmagneten konstruiert sind.

5. Verfahren zur Bereitstellung überlagerter Magnetresonanz- und plenoptischer Abbildungen, wobei das Verfahren umfasst:
Bereitstellen einer MRT-Vorrichtung nach Anspruch 1;
Platzieren eines Objekts in einem Gesichtsfeld der MRT-Vorrichtung;
Anwenden der Magnetresonanz-Bildgebungsmittel zum Produzieren einer durch einen Parallaxenwinkel und eine Feldtiefe gekennzeichneten Magnetresonanzabbildung;
Anwenden der plenoptischen Kamera zum Produzieren, zeitgleich mit der Magnetresonanzabbildung, einer durch einen Parallaxenwinkel und eine Feldtiefe gekennzeichneten plenoptischen Abbildung;
Wiedergeben der Magnetresonanz- und plenoptischen Abbildungen; und
Überlagern dieser Magnetresonanz- und plenoptischen Abbildungen.

6. Verfahren nach Anspruch 5, wobei der Schritt des Produzierens einer plenoptischen Abbildung das Produzieren einer plenoptischen Abbildung mit einem Parallaxenwinkel, der identisch zu dem Parallaxenwinkel der Magnetresonanzabbildung ist, umfasst.

7. Verfahren nach Anspruch 5, wobei der Schritt des Produzierens einer plenoptischen Abbildung das Produzieren einer plenoptischen Abbildung mit einer Feldtiefe, die identisch zu der Feldtiefe der Magnetresonanzabbildung ist, umfasst.

8. Verfahren nach Anspruch 5, wobei der Schritt der Bereitstellung einer MRT-Vorrichtung das Bereitstellen einer MRT-Vorrichtung, die aus Permanentmagneten konstruierte Magnetpole aufweist, umfasst.

## Revendications

1. Appareil d'imagerie par résonance magnétique pour procurer des images plénoptiques et de résonance magnétique superposées, ledit appareil comprenant :
des pôles magnétiques (220) pour créer un champ magnétique homogène ;
un jeu de bobines RF pour générer une impulsion d'excitation RF dans le volume de formation d'image (230) de l'appareil, et pour acquérir des signaux de résonance magnétique qui sont dus à l'impulsion d'excitation RF ;
des moyens d'imagerie par résonance magnétique pour transformer lesdits signaux de résonance magnétique en une image de résonance magnétique ;
un appareil photographique à champ de lumière, c'est-à-dire plénoptique (100), configuré pour obtenir les images plénoptiques de manière concourante avec lesdites images de résonance magnétique ; et
des moyens de superposition pour superposer lesdites images plénoptiques et lesdites images de résonance magnétique.

2. Appareil d'imagerie par résonance magnétique selon la revendication 1, dans lequel lesdites images de résonance magnétique et lesdites images plénoptiques sont **caractérisées par** un angle parallactique identique.

3. Appareil d'imagerie par résonance magnétique selon la revendication 1, dans lequel lesdites images de résonance magnétique et lesdites images plénoptiques sont **caractérisées par** une profondeur de champ identique.

4. Appareil d'imagerie par résonance magnétique selon la revendication 1, dans lequel lesdits pôles magnétiques (220) sont construits à partir d'aimants permanents.

5. Procédé pour procurer des images plénoptiques et de résonance magnétique superposées, ledit procédé comprenant le fait de :
procurer un appareil d'imagerie par résonance magnétique selon la revendication 1 ;
placer un objet à l'intérieur du champ de vision dudit appareil d'imagerie par résonance magnétique ;
utiliser ledit moyen d'imagerie par résonance magnétique pour obtenir une image de résonance magnétique **caractérisée par** un angle parallactique et une profondeur de champ ;
utiliser ladite caméra plénoptique pour obtenir, de manière concourante avec ladite image de résonance magnétique, une image plénoptique **caractérisée par** un angle parallactique et une profondeur de champ ;
obtenir un rendu sous la forme d'images plénoptiques et de résonance magnétique ; et
superposer lesdites images plénoptiques et de résonance magnétique.

6. Procédé selon la revendication 5, dans lequel ladite étape de production d'une image plénoptique comprend la production d'une image plénoptique possédant un angle parallactique identique audit angle parallactique de ladite image de résonance magnétique.

7. Procédé selon la revendication 5, dans lequel ladite étape de production d'une image plénoptique comprend la production d'une image plénoptique possédant une profondeur de champ identique à ladite profondeur de champ de ladite image de résonance magnétique.

8. Procédé selon la revendication 5, dans lequel ladite étape dans laquelle on procure un appareil d'imagerie par résonance magnétique comprend le fait de procurer un appareil d'imagerie par résonance magnétique comprenant des pôles magnétiques construits à partir d'aimants permanents.
